# EUROPEAN PATENT SPECIFICATION

(11) **EP 0 705 082 B1**
(45) Date of publication and mention of the grant of the patent: **18.09.2002**
(21) Application number: 94917506.1
(22) Date of filing: 03.06.1994
(51) Int. Cl.: A61F 5/04, A61B 17/62

(54) **DISTRACTOR MECHANISM FOR EXTERNAL FIXATION DEVICE**
STRECKMECHANISMUS FÜR EXTERNE FIXIERVORRICHTUNG
MECANISME DISTRACTEUR POUR DISPOSITIF DE FIXATION EXTERNE

(30) Priority: 10.06.1993 US 75179; 02.02.1994 US 190654
(43) Date of publication of application: 10.04.1996
(73) Proprietor: TEXAS SCOTTISH RITE HOSPITAL FOR CRIPPLED CHILDREN, Dallas, TX 75219 (US)
(72) Inventor: ROSS, John, David, Jr., Ovilla, TX 75154 (US); SAMCHUKOV, Mikhail, L., Dallas, TX 75006 (US); BIRCH, John, G., Dallas, TX 75243 (US)
(74) Representative: Weydert, Robert
(86) International application number: PCT/US94/06213
(87) International publication number: WO 94/028829

(56) References cited:
- EP-A- 0 369 017
- SU-A- 367 858
- SU-A- 611 612
- SU-A- 858 797
- US-A- 2 055 024
- US-A- 4 541 422
- US-A- 4 615 338
- US-A- 4 978 348

## Description

### 1. Field of the Invention

The present invention relates to external fixation devices, and in particular, to distractor mechanisms for external fixation devices.

### 2. Description of the Related Art

External fixation of bone fractures is well known in the art. Many different external fixation devices have been developed, virtually all of which in one form or another use multiple transverse fixation wires or pins which extend through, or are embedded in, respectively, the bone and soft tissue surrounding the bone, and connect to various types of supporting elements, such as rings, half-rings, arches or bars.

Aside from providing stable fixation of bone fragments to promote proper fracture healing, external fixation devices also provide means for transporting the bone fragments in order to correct length discrepancies or angular deformities of the bone. One of the more common external fixation devices used for such purposes, often referred to as the Ilizarov External Fixator, includes three basic elements: multiple rings (or arches) disposed coaxially about the bone segments to be fixated; transverse wires or pins for fixating the bone segments to the rings (or arches); and distractor mechanisms.

Hence, for linear distraction, a typical basic assembly includes proximal and distal rings, or ring sets, connected by three or four distractor mechanisms.

A conventional distractor mechanism for linear distraction consists of a threaded rod which is fastened to each of the rings by the use of double nut assemblies, i.e. nuts which are threaded onto the rod and tightened against either side of each ring, such as disclosed in SU-A-858797 on which the two-part form of the independent claim is based.

The current conventional external fixator elements (distractors) including SU-A-858797 present a number of problems. First, because the nut assemblies must be loosened to permit adjustment and the connection points for the distractor mechanisms, i.e. the holes in the rings and hinge members, must be slightly larger than the diameter of the threaded component (rods and bolts), the resulting assembly is always unstable when the nut assemblies are loosened. In other words, during installation, adjustment or distraction, a certain amount of mechanical "free play" will be present. During the treatment period of the patient, this can and often does cause pain.

Currently, there are two methods used in seeking to compensate for this problem. One method is to loosen and retighten all hinge and distractor nuts before and after each linear distraction maneuver. This can be effective, but is time consuming and frustrating for the patient. Plus, the torque which must be applied to loosen and retighten the hardware can adversely affect the healing process and cause pain for the patient. The second method involves the use of nylock nuts in the distractor mechanisms to achieve some degree of radial stability by imposing an axial preload on the rotating elements. The mechanical "free play" is still there, but is damped by frictional forces from the fasteners. However, even when the distractor is precisely adjusted, the potential for an unstable frame is still present due to the oversized hole diameters.

A second problem arises during each distraction maneuver. Each incremental distraction takes a great deal of time, and the procedure is generally too complex for young patients to perform reliably. With so many components, i.e. plates, rods, bolts and nuts, to loosen, turn and retighten, it is difficult for the patient to remember the adjustment sequence and maintain a consistent distraction rate. Moreover, if any of the nuts are accidentally left untightened, instability, and therefore, pain and delayed healing, can result.

Other examples of the aforementioned conventional external fixator assemblies introducing "play" into the system during installation, adjustment or distraction are disclosed in SV-A-611612 and US-A-2,055,024.

Accordingly, the object of the invention is to provide an external fixation device with a distraction mechanism which allows adjustable distraction while simultaneously providing a rigid structure, i.e. both radial and axial stability of the overall assembly.

### SUMMARY OF THE INVENTION

To achieve this object there is provided in accordance with the invention an external fixator assembly for rigidly immobilizing a bone fracture during linear distraction of the bone segments, comprising first and second external fixator frame members and at least one distractor mechanism for connecting to said first and second external fixator frame members and rigidly disposing said second external fixator frame member at a separation distance from said first external fixator frame member, said distractor mechanism including a single-threaded rod fixedly fastened to said second external fixator frame member, and a rotatable connector connecting said single-threaded rod to said first external fixator frame member to permit linear adjustement of said separation distance between the frame members, wherein said rotatable connector is a fastener assembly interconnecting the distractor mechanism to the first external fixator frame member, said fastener assembly comprising a pair of threaded nuts, disposed on opposing sides of said first external fixator frame member with said single-threaded rod extending and threading therethrough so that the first and second external fixator frame members and the distractor mechanism form a rigid structure, characterized in that said nuts are plastic nuts aligned for simultaneous interfacing with and rotation by a double wrench so that the rigidity of said rigid structure is maintained during the adjusting of said separation distance.

These and other features and advantages of the present invention will be understood upon consideration of the following detailed description of the invention and the accompanying drawings.

### BRIEF DESCRIPTION OF THE DRAWINGS

Figure 1 illustrates an external fixator of a linear distraotor assembly which does not form part of the invention but is useful for understanding the invention.

Figure 2 illustrates a cross-section (along line 2-2) of one of the linear distractor mechanisms of figure 1.

Figures 3A and 3B illustrate a ratchet assembly which can be used on the linear distraotor mechanisms of figure 1.

Figure 4 illustrates an external fixator with a linear distractor assembly in accordance with the present invention.

### DETAILLED DESCRIPTION OF THE INVENTION

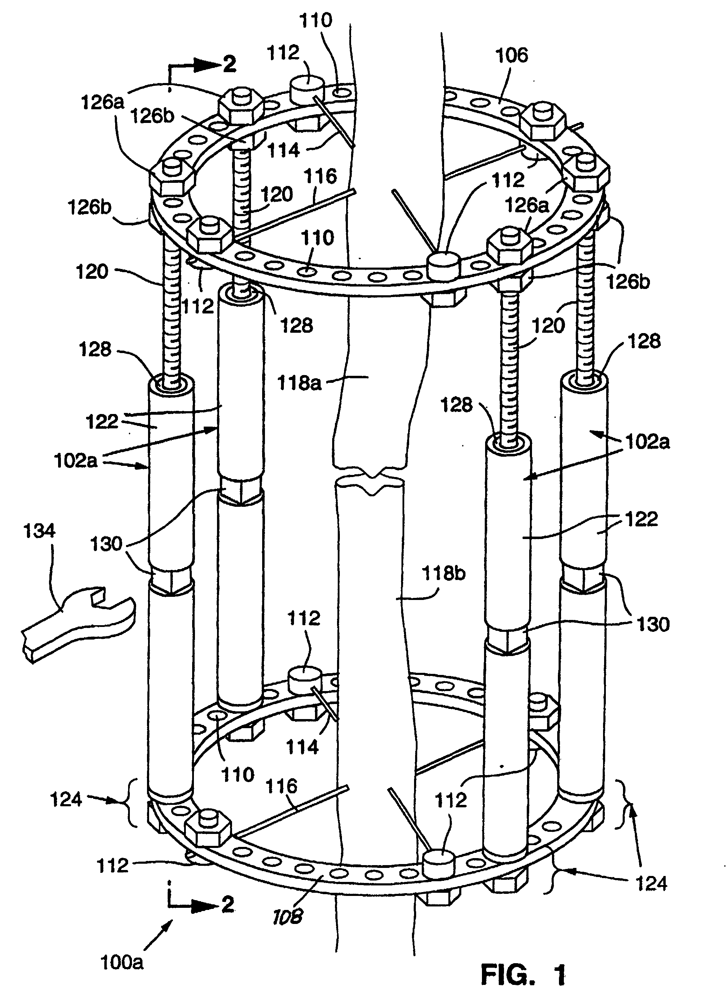

Referring to figure 1, an external fixator assembly 100a for performing linear distraction includes linear distractor mechanisms 102a, and upper 106 and lower 108 external fixator frame members, or rings. Each ring 106, 108 includes multiple, spaced holes 110, a number of which are used for mounting fastener assemblies 112 (discussed further below) for fastening transverse wires 114 and/or pins 116. These wires 114 and/or pins 116 pass through or are anchored into, respectively, the bone segments 118a, 118b which are to be externally fixated with the fixator assembly 100a.

Each distractor mechanism 102a includes a single-threaded rod 120 which is coaxially mated with an internally like-threaded, plastic insert 128 mounted within a rotatable sleeve 122. The rod 120 is fastened to the upper ring 106 by extending it through one of the holes 110 and having two nuts 126a, 126b tightened against opposing sides of the ring 106. The sleeve 122 is rotatively connected to the lower ring 108 by way of a rotatable connector 124 (discussed further below). At approximately the midway point of the sleeve 122, is a recessed, square portion 130 which can be used as a tool interface for mating with a tool (e.g. a wrench 134) for rotating the sleeve 122.

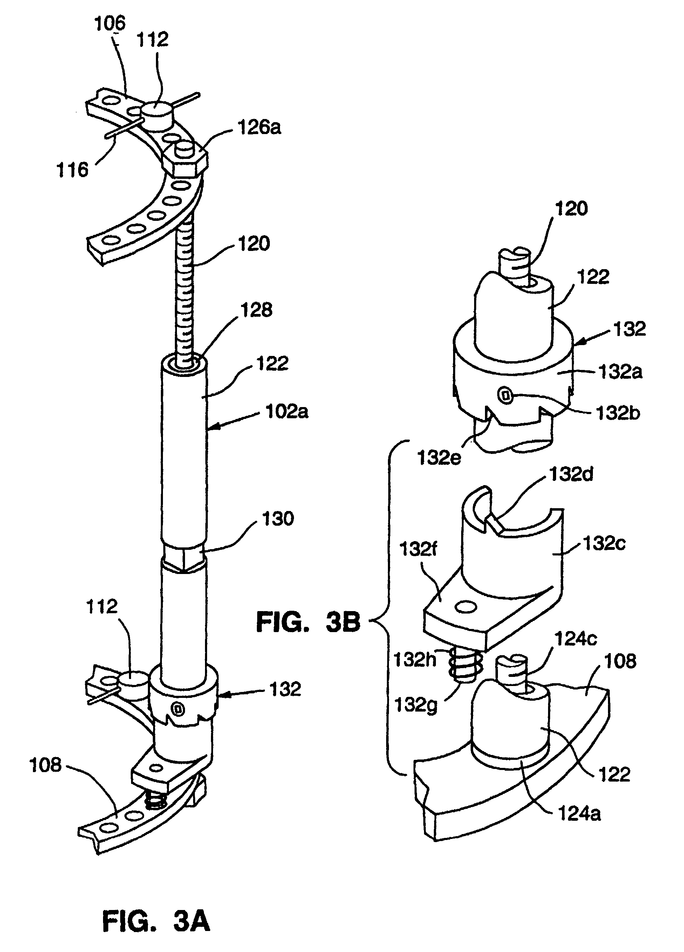

The distractor mechanisms 102a can also include a ratchet assembly 132 (as shown in figures 3A and 3B, and discussed further below). This ratchet assembly 132 prevents rotation of the sleeve 122 in a selected direction.

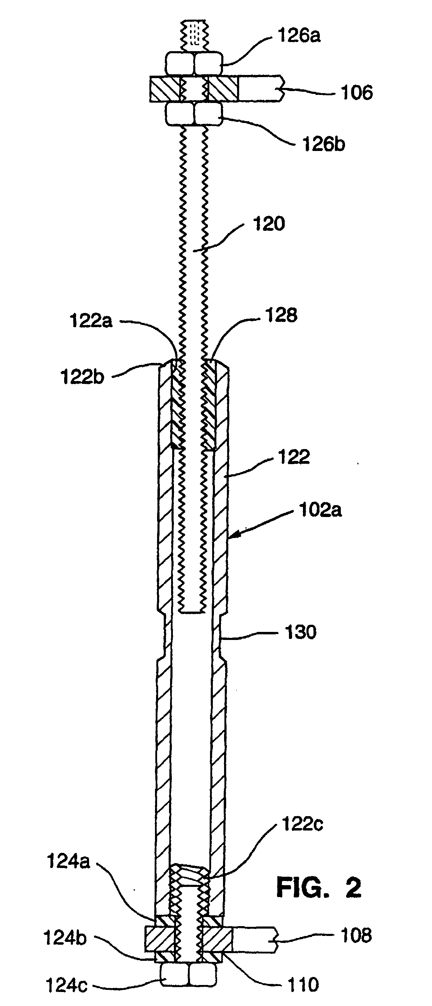

Referring to figure 2, the construction of a linear distractor mechanism 102a can be understood. The metal-to-plastic interface of the threaded rod 120 and like-threaded plastic insert 128 can be seen in cross-section. The plastic insert 128 is pressed into the end 122a of the rotatable sleeve 122 which is counterbored, and is prevented from rotating within or being pulled out from the sleeve 122 by crimping the tip 122b of the sleeve 122 (e.g. in a pyramidal shape) after the insert 122 has been pressed therein.

Referring still to Figure 2, the rotatable connector 124 can be better understood. The rotatable sleeve 122 is connected to the bottom ring 108 by placing plastic washers 124a and 124b on opposite sides of the ring 108, and passing a bolt 124c through the ring hole 110 and plastic washers 124a, 124b to be mated with internal locking threads 122c (e.g. screw thread inserts produced under the trademark HELI-COIL®) of the sleeve 122. The bolt 124c is then tightened down within the sleeve 122 and against the plastic washers 124a, 124b to eliminate any axial free play between the ring 108 and sleeve 122. These internal locking threads 122c prevent the bolt 124c from turning independently from the sleeve 122. This prevents the union of the bolt 124c and sleeve 122 from becoming overly tight (which would inhibit rotation of the sleeve 122) or loose (which would introduce undesirable axial free play between the ring 108 and sleeve 122). The plastic washers 124a, 124b are compressed by the tightened bolt 124c and significantly reduce friction, thereby assisting in setting and maintaining an appropriate preload between the metal bolt 124c, rotatable sleeve 122 and ring 108.

Due to the metal-to-plastic interfaces of the sleeve 122, plastic washer 124a and ring 108, and of the bolt 124c, plastic washer 124b and ring 108, the sleeve 122 can be selectively rotated while simultaneously forming and maintaining a rigid structure with respect to the lower ring 108. This rigidity is further maintained by the metal-to-plastic interface of the threaded rod 120 and plastic insert 128, as the metal rod 120 remains fixed, and therefore rigid, with respect to the upper ring 106.

The threaded rod 120 has a hexagonal socket 120a in the end to facilitate rotation of the rod 120 into or out of the sleeve 122 by use of a conventional hexagonal key wrench (not shown). Such adjustments are often necessary when initially adjusting or attaching a linear distractor mechanism 102a to the rings 106, 108, since the interface between the plastic insert 128 and the threaded rod 120 is sufficiently tight as to require more than finger pressure on the rod 120 to rotate it into and out of the sleeve 122. However, the larger diameter of the rotatable sleeve 122 provides sufficiently greater leverage that ordinary finger pressure is sufficient to rotate the sleeve 122 on the rod 120 once the rod 120 is secured to the ring 106 with the nuts 126a, 126b.

Referring to Figures 3A and 3B, the ratchet assembly 132 can be better understood. The ratchet assembly 132 includes a circular collar 132a, which is fixed about the rotatable sleeve 122 via compression with a hexagonal socket set screw 132b. This circular collar 132a sits atop an arcuate collar 132c which is also coaxial with the sleeve 122. This arcuate collar 132c has a unidirectionally sloped tooth 132d which engages similarly shaped notches 132e in the lower edge of the circular collar 132a. A flange 132f extending from the arcuate collar 132c has a downwardly extending pin 132g which engages a hole 110 in the lower ring 108. A helical spring 132h wound coaxially about this pin 132g upwardly biases the arcuate collar 132c. Thus, as the sleeve 122, and therefore the circular collar 132a, is rotated, the mating tooth 132d and notches 132e allow the sleeve 122 to rotate in one direction only.

This ratchet assembly 132 advantageously allows the sleeve 122 to be rotated in calibrated amounts, e.g. by listening for the "clicks" made as the tooth 132d snaps into place when engaging one of the notches 132e as the sleeve 122 is rotated. This allows the user of the external fixator 100a to make calibrated adjustments of the distractor mechanism 102a without the aid of tools and without the need for counting fractional revolutions of the sleeve 122. Instead, the number of clicks can be counted to determine if and when sufficient adjustment has been made.

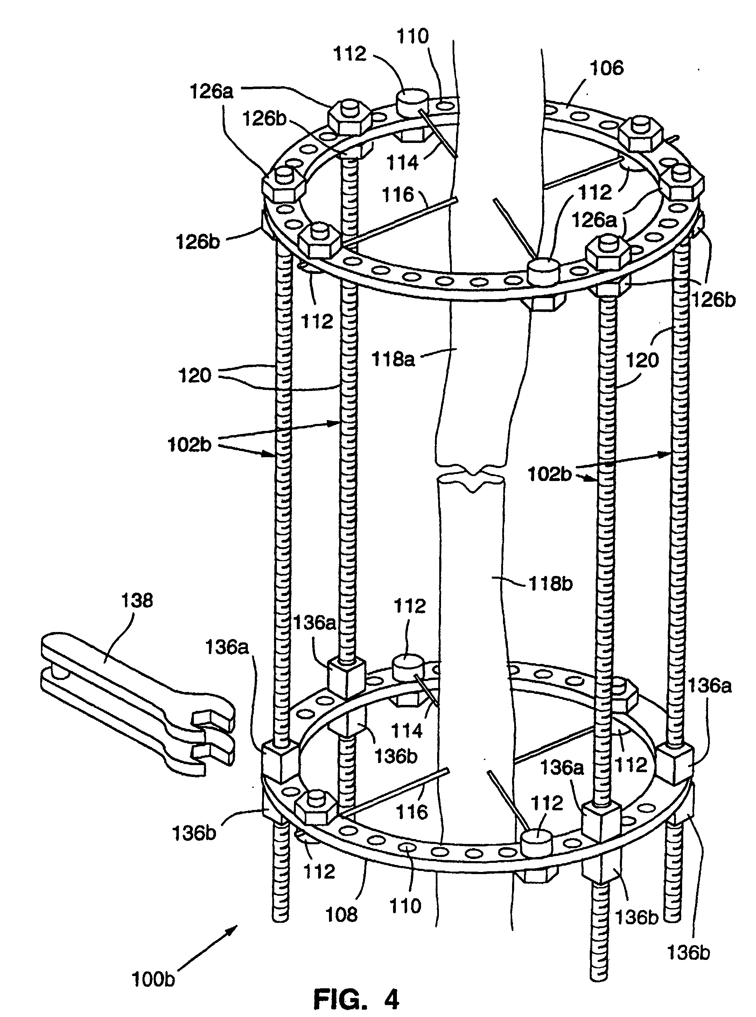

Referring to Figure 4, an external fixator assembly 100b for performing linear distraction with a linear distractor assembly in accordance with the present invention includes linear distractor mechanisms 102b of an alternative type, and upper 106 and lower 108 external fixator frame members, or rings, as discussed above. This type of linear distractor mechanism 102b is particularly useful in those cases where the initial, or starting, distance between the rings 106, 108 and the amount of lengthening are small. In such cases, the single-threaded rod 120 is secured to one ring 106 with two nuts 126a, 126b, as discussed above, and is passed through a hole 110 in the other ring 108 and is secured thereto with two plastic prevailing torque lock nuts 136a, 136b which are tightened against opposing sides of the ring 108. The faces of the plastic nuts 136a, 136b are aligned for simultaneous interfacing with and turning in the desired direction by a double wrench 138.

The threaded rods 120 (figures 1 and 4) are metal, preferably stainless steel. The rotatable sleeve 122 (figure 1) is metal, preferably aluminium. The various plastic inserts 128 (figure 1), plastic washers 124a, 124b (figure 2), and plastic nuts 136a, 136b (figure 4) are preferably nylon (e.g. Nylon 6/6) polyphenylene sulfide, polyamid or polyimid. However, other plastics having similar resiliency qualities can be used as well.

It should be recognized that the terms "upper" and "lower" with respect to the two external fixator rings 106, 108 have been used for purposes of convenience only, and are not to be construed as limitations upon their actual location or orientation with respect to one another.

## Claims

1. External fixator assembly for rigidly immobilizing a bone fracture during linear distraction of the bone segments, comprising first and second external fixator frame members (108, 106) and at least one distractor mechanism (102b) for connecting to said first and second external fixator frame members (108, 106) and rigidly disposing said second external fixator frame member (106) at a separation distance from said first external fixator frame member (108), said distractor mechanism (102b;) including a single-threaded rod (120) fixedly fastened to said second external fixator frame member (106), and a rotatable connector (124) connecting said single-threaded rod (120) to said first external fixator frame member (108) to permit linear adjustement of said separation distance between the frame members (108, 106), wherein said rotatable connector (124) is a fastener assembly interconnecting the distractor mechanism (102b) to the first external fixator frame member (108), said fastener assembly comprising a pair of threaded nuts (136a, 136b), disposed on opposing sides of said first external fixator frame member (108) with said single-threaded rod (120) extending and threading therethrough so that the first and second external fixator frame members (108, 106) and the distractor mechanism (102b) form a rigid structure, **characterized in that** said nuts (136a, 136b) are plastic nuts (136a, 136b) aligned for simultaneous interfacing with and rotation by a double wrench (138) so that the rigidity of said rigid structure is maintained during the adjusting of said separation distance.

2. External fixator assembly of claim 1, **characterized in that** said plastic nuts (136a, 136b) are prevailing torque lock nuts.

## Patentansprüche

1. Externe Fixiervorrichtung zum starren Unbeweglichmachen eines Knochenbruches während dem linearen Strecken der Knochenbruchstücke, mit einem ersten und einem zweiten äusseren Fixierrahmenteil (108, 106) und wenigstens einem Streckmechanismus (102a; 102b) zum Miteinanderverbinden des ersten und des zweiten äusseren Fixierrahmenteiles (108, 106) und zum starren Einstellen des zweiten äusseren Fixierrahmenteiles (106) in einem Abstand von dem ersten äusseren Fixierrahmenteil (108), wobei der Streckmechanismus (102a; 102b) eine eingängige Gewindestange (120) aufweist, die an dem zweiten äusseren Fixierrahmenteil (106) starr befestigt ist, und einen Drehverbinder (124) aufweist, der die eingängige Gewindestange (120) mit dem ersten äusseren Fixierrahmenteil (108) verbindet, um eine lineare Einstellung des Abstandes zwischen den Rahmenteilen (108, 106) zu erlauben, wobei der Drehverbinder (124) eine Befestigungseinrichtung ist, welche den Streckmechanismus (102a; 102b) mit dem ersten äusseren Fixierrahmenteil (108) verbindet, und die Befestigungseinrichtung zwei Gewindemuttern (136a, 136b) aufweist, die sich auf gegenüberliegenden Seiten des ersten äusseren Fixierrahmenteiles (108) befinden, und die eingängige Gewindestange (120) durch die Gewindemuttern ragt und in diese eingeschraubt ist, damit der erste und der zweite äussere Fixierrahmenteil (108, 106) und der Streckmechanismus (102a; 102b) eine starre Struktur bilden, **dadurch gekennzeichnet, dass** die Muttern (136a, 136b) Kunststoffmuttern (136a, 136b) sind, die ausgelegt sind, um durch einen doppelten Schraubschlüssel (138) gleichzeitig erfasst und gedreht zu werden, damit die Steifheit der starren Struktur während dem Einstellen des Abstandes aufrechterhalten wird.

2. Externe Fixiervorrichtung nach Anspruch 1, **dadurch gekennzeichnet, dass** die Kunststoffmuttern (136a, 136b) selbstsichernde Blockiermuttern sind.

## Revendications

1. Dispositif de fixation externe pour immobiliser rigidement une fracture d'os pendant la distraction linéaire des fragments de l'os, comportant un premier et un second élément (108, 106) formant un cadre de fixation externe et au moins un mécanisme de distraction (102a ; 102b) pour connecter lesdits premier et second éléments formant le cadre de fixation externe (108, 106) et pour positionner ledit second élément du cadre de fixation externe (106) rigidement en une distance de séparation du premier élément du cadre de fixation externe (108), ledit mécanisme de distraction (102a ; 102b) incluant une tige (120) à filet unique attachée rigidement au second élément du cadre de fixation externe (106), et un connecteur rotatif (124) connectant la tige (120) à filet unique au premier élément du cadre de fixation externe (108) pour permettre un ajustement linéaire de ladite distance de séparation entre les éléments formant le cadre (108, 106), ledit connecteur rotatif (124) étant un ensemble de fixation interconnectant le mécanisme de distraction (102a ; 102b) audit premier élément du cadre de fixation externe (108), ledit ensemble de fixation comportant une paire d'écrous filetés (136a, 136b) disposés sur les côtés opposés du premier élément du cadre de fixation externe (108) et la tige (120) à filet unique s'étendant par ces écrous tout en étant reçue par engagement vissé dans ces écrous de sorte que le premier et le second élément du cadre de fixation externe (108, 106) et le mécanisme de distraction (102a ; 102b) forment une structure rigide, **caractérisé en ce que** lesdits écrous (136a, 136b) sont des écrous en matière plastique (136a, 136b) alignés en vue d'être engagés et tournés simultanément par une clé double (138) de sorte que la rigidité de ladite structure rigide soit maintenue pendant le réglage de ladite distance de séparation.

2. Dispositif de fixation externe selon la revendication 1, **caractérisé en ce que** les écrous en matière plastique (136a, 136b) sont des écrous de blocage indeserrables.
